# EUROPÄISCHE PATENTSCHRIFT

(11) **EP 1 310 727 B1**
(45) Veröffentlichungstag und Bekanntmachung des Hinweises auf die Patenterteilung: **21.06.2006**
(21) Anmeldenummer: 02102526.7
(22) Anmeldetag: 04.11.2002
(51) Int. Cl.: F16M 11/12, G02B 7/00, G02B 21/26, A61B 19/00, F16M 11/10

(54) **Operations-Mikroskop**
Surgical microscope
Microscope chirurgical

(30) Priorität: 13.11.2001 DE 10155719
(43) Veröffentlichungstag der Anmeldung: 14.05.2003
(73) Patentinhaber: Leica Microsystems (Schweiz) AG, 9435 Heerbrugg (CH)
(72) Erfinder: Metelski, Andrzej, 8590 Romanshorn (CH)
(74) Vertreter: Rosenich, Paul

(56) Entgegenhaltungen:
- EP-A- 0 700 665
- EP-A- 0 791 339
- DE-A- 4 226 608
- DE-A- 4 416 178
- GB-A- 1 431 653
- JP-A- 53 087 247
- US-A- 5 861 983
- PATENT ABSTRACTS OF JAPAN Bd. 1998, Nr. 01, 30. Januar 1998 (1998-01-30) -& JP 09 224956 A (MITAKA KOKI CO LTD), 2. September 1997 (1997-09-02)

## Beschreibung

Die Erfindung betrifft ein Operations-Mikroskop mit einem Ständer und einem mit dem Ständer beweglich verbundenen, gegenüber dem Ständer beweglich gelagerten Optikträger, wobei der Optikträger im Bereich seines Schwerpunktes gegenüber einem Schwenkträger des Ständers um wenigstens eine Achse schwenkbar gelagert ist.

Operations-Mikroskope müssen sehr beweglich und ohne großen Kraftaufwand rasch verstellbar sein. Damit sich das Mikroskop in einer einmal eingestellten Position nicht selbsttätig verstellt, müssen die auftretenden Kräfte und Momente in jeder Lage ausgeglichen sein. Sind sie nicht ausgeglichen, so müssen Bremsen oder Stützeinrichtungen vorhanden sein, die wiederum das Gesamtgewicht nachteilig erhöhen, ohne optimal zu balancieren (Hysterese). Jedoch auch dann, wenn Bremsen vorhanden sind, sollen die Kräfte und Momente ausgeglichen sein, um ein "federleichtes" Bewegen des Mikroskops bei gelösten Bremsen zu ermöglichen.

Operationsmikroskope werden beim Einsatz mit einer Vielfalt von unterschiedlichem Zubehör (z.B. Tuben, Verlängerungen, Filter, Vorsatzlinsen etc.) verwendet. Dieses Zubehör hat jedoch in vielen Fällen eine Verlagerung des Gesamtschwerpunktes zur Folge. Dadurch ist das System nicht mehr im Gleichgewicht und muss neu ausbalanciert werden. Dieses Ausbalancieren kann beispielsweise durch das Anbringen, Entfernen oder Verschieben zusätzlicher Ausgleichsgewichte erfolgen. Dieses Verfahren ist jedoch sehr zeitaufwändig und ergibt zudem erhöhte Kräfte und Momente am Ständer.

Aus der DE 42 14 858 C1 ist ein Stativ für ein medizinisches Röntgengerät bekannt, das einen C-förmigen Bogen trägt. An einem Ende des Bogens ist ein Röntgenstrahler und am anderen Ende ein Bildverstärker angeordnet ist.

Außerdem ist ein Optikträger bekannt, der nur einseitig (links vom Mikroskop) am Schwenkträger angelenkt ist. Damit ist der Bereich der Achse im Schwerpunkt rechts vom Mikroskop frei, um dort Zubehör oder Zusatzeinrichtungen zu montieren.

Wegen der linksseitigen Anbindung entfällt nachteiligerweise die Möglichkeit, auch hier Zubehör anzubringen.

Der Erfindung liegt die Aufgabe zugrunde, eine Vorrichtung zu schaffen, welche ein einfaches und rasches Ausbalancieren des Operations-Mikroskops ermöglicht und die Nachteile der bekannten Lösungen, insbesondere die Montage von zusätzlichen Gewichten oder einseitige Anbindungen vermeidet.

Diese Aufgabe wird erfindungsgemäß durch die im Kennzeichen des Patentanspruchs 1 angegebenen Merkmale gelöst. Vorteilhafte Weiterbildungen der Erfindung sind Gegenstand der Unteransprüche.

Die eigentliche Führung des Optikträgers befindet sich somit ausserhalb der virtuellen Schwenkachse. Da sich der Schwerpunkt des Optikträgers häufig im Bereich des optischen Systems befindet, ist es somit möglich, die virtuelle Schwenkachse genau in den Schwerpunkt des Optikträgers zu legen, ohne dass dabei das optische System durch reelle Achsen oder dgl. beeinträchtigt wird. Dies erlaubt optische Zusatzeinrichtungen auch im Bereich der Achse mit dem Mikroskop zu verbinden, und dies auf beiden Seiten des Mikroskops, ohne dass ein zusätzlicher Gewichtsausgleich (Zusatzgewicht) am Optikträger montiert werden muss. Gegenüber dem bekannten Aufbau des Optikträgers der Anmelderin (WO 97 13 997 A1) wird somit insgesamt das Gewicht des Optikträgers reduziert. Daraus folgt eine Reduktion des gesamten Trägheitsmoments, respektive des Gewichts, beim Gesamtaufbau. Eine leichtgängige Einstellbarkeit ist die Folge.

Das bogensegmentförmige Führungselement wirkt zweckmäßigerweise mit einem Führungswagen oder Führungskäfig zusammen. Diese beiden Elemente bilden zusammen die eigentliche Führung. Das für die relative Beweglichkeit erforderliche Führungsspiel kann beispielsweise durch die Fabrikationstoleranzen definiert oder einstellbar sein.

Eine vorteilhafte Ausführung der Erfindung besteht darin, dass das bogensegmentförmige Führungselement mit dem Optikträger in wenigstens einer Raumachse verstellbar verbunden ist. Diese Verbindung kann über ein Zwischenglied erfolgen.

Die Achse kann in der Ausgangslage des Optikträgers horizontal oder vertikal verlaufen.

Die Verstellung des bogensegmentförmigen Führungselements erfolgt vorteilhaft über lineare Schlitten. Solche Schlitten ermöglichen eine genaue Führung und sind in der Lage, Kräfte und auch eventuell auftretende Kippmomente aufzunehmen.

Bei Änderungen des Zubehörs am Mikroskop verschiebt sich der Gesamtschwerpunkt des Optikträgers sehr oft wenigstens zweidimensional. Zum Ausbalancieren des Systems sind daher zweckmäßigerweise zwei im Wesentlichen senkrecht zueinander angeordnete Schlitten vorgesehen. Somit ist das Verschieben des bogensegmentförmigen Führungselementes und somit auch der virtuellen Achse möglich. Die virtuelle Achse kann somit einfach in den Schwerpunkt des Optikträgers gebracht werden.

Für ein rasches und präzises Ausbalancieren des Optikträgers erfolgt die Verstellung des bogensegmentförmigen Führungselementes vorteilhaft mittels Gewindespindeln. Solche Gewindespindeln sind bevorzugt selbsthemmend. Eine zusätzliche Klemmung ist daher in der Regel nicht notwendig.

Im Rahmen der Erfindung liegen auch motorische Antriebe für die Gewindespindeln, die auch ferngesteuert und/oder mittels automatischer Ansteuerung geregelt werden.

Bei einem nicht ausbalancierten System, beispielsweise beim Wechseln des Zubehörs, können am bogensegmentförmigen Führungselement größere Kräfte und Momente auftreten. Um diese wenigstens teilweise aufzunehmen, ist am bogensegmentförmigen Führungselement ein Bremssystem vorgesehen. Das Bremssystem kann zum Verstellen des Optikträgers ganz oder teilweise lösbar sein.

Das Bremssystem weist vorteilhaft eine mit dem bogensegmentförmigen Führungselement zusammenwirkende, feststellbare Zange auf. Eine solche Zange kann das Führungselement, wie bei einer Scheibenbremse, von beiden Seiten her umgreifen und bei Bedarf festklemmen. Andere, auch lineare Bremseinrichtungen, liegen ebenso im Bereich der Erfindung.

Damit das Mikroskop durch leichtes Berühren nicht unbeabsichtigt verstellt wird, andererseits jedoch bei Bedarf auch bei angezogener Bremse in seiner Lage verändert werden kann, weist das Bremssystem eine regulier- und einstellbare Bremskraft auf. Diese Bremskraft kann durch Federelemente, Elektromagnete oder eine Kombination von beiden erzeugt werden.

Das bogensegmentförmige Führungselement sollte aus statischen Gründen meistens in einer außerhalb des Gesamtschwerpunktes liegenden, senkrecht zur Achse verlaufenden Ebene angeordnet sein. Dadurch entstehen gegebenenfalls Kippmomente, welche sich auf die Stabilität des Systems negativ auswirken könnten. Um solche Kippmomente zu vermeiden, sind gemäß einer Weiterbildung der Erfindung, pro Schwenkachse jeweils zwei parallel zueinander angeordnete bogensegmentförmige Führungselemente vorgesehen. Alternativ oder zusätzlich kann auch eine Verstelleinrichtung für den Optikträger vorgesehen sein, der den Optikträger entlang der Achse seitlich verschiebbar macht (Y-Verstellung).

Um ein leichtgängiges Verstellen des Mikroskops zu ermöglichen, sollte die Reibung der Führungselemente gering sein. Es ist daher zweckmäßig, dass das Führungselement als gebogener Kugelführungsschlitten ausgebildet ist. Solche Kugelführungsschlitten funktionieren im Prinzip wie Wälzlager und weisen einen sehr geringen Rollwiderstand auf. Vorzugsweise können dazu handelsübliche Lagerelemente verwendet werden. Sollte hingegen eine gewisse Grundreibung wegen ihrer Bremswirkung erwünscht sein, so kann anstelle von Wälzlagern eine Gleitlagerführung eingesetzt werden.

Vorzugsweise sind zwei parallel zueinander angeordnete Führungselemente vorgesehen. Damit wird die Beanspruchung durch die auftretenden Kräfte und Momente auf die einzelnen Führungselemente gering gehalten.

Die Erfindung ist in Ausführungsbeispielen dargestellt und wird mit den nachfolgenden schematischen näher erläutert. Es zeigen:
- Fig. 1: ein erfindungsgemäßes Operationsmikroskop mit einem Ständer,
- Fig. 2: einen Ausschnitt des in Fig. 1 dargestellten Mikroskops, in vergrößertem Maßstab,
- Fig. 3: eine Variante des Mikroskops mit zwei parallel zueinander angeordneten Führungselementen.

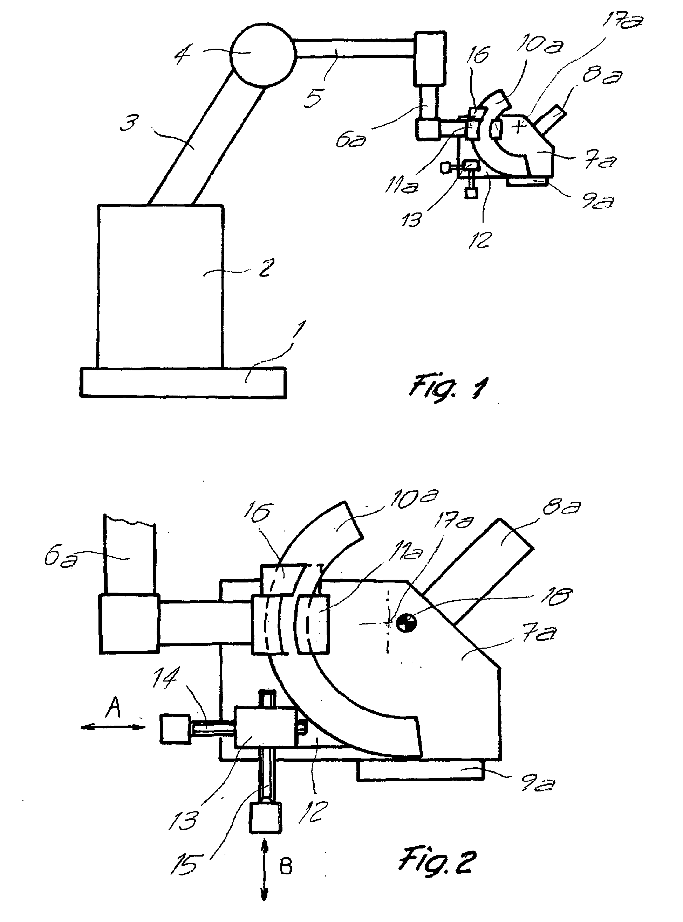

Das aus Fig. 1 ersichtliche Operations-Mikroskop weist einen Ständer auf, der im Wesentlichen aus einem Ständerfuß 1, einer Ständersäule 2 und einem in der Ständersäule 2 gelagerten Ausleger 3 besteht. Ein Tragarm 5 ist über ein Gelenk 4 mit dem Ausleger 3 verbunden. Am freien Ende des Tragarms 5 hängt ein in einer horizontalen Ebene beweglicher Schwenkträger 6a. Ein Optikträger 7a ist in mehreren Achsen beweglich mit dem Schwenkträger 6a verbunden. Am Optikträger 7a sind als auswechselbare Zubehörteile ist exemplarisch ein Tubus 8a sowie ein Objektiv 9a dargestellt.

Unter dem Begriff "Ständer" im Sinne der Erfindung sind alle jene Halterungen zu verstehen, die einen Schwenkträger gegenüber seiner Umgebung beweglich halten, wie dies beispielsweise für Wand-, Boden-, Decken-Stative zutrifft. Der Aufbau des Ständers ist für die Erfindung nicht wesentlich.

Die Verbindung des Optikträgers 7a mit dem Schwenkträger 6a erfolgt über ein bogensegmentförmiges Führungselement 10a sowie einen damit zusammenwirkenden Führungswagen 11a. In diesem Ausführungsbeispiel ist das Führungselement 10a mit dem Optikträger 7a und der Führungswagen 11a mit dem Schwenkträger 6a verbunden. Im Prinzip kann dies jedoch auch gerade umgekehrt sein.

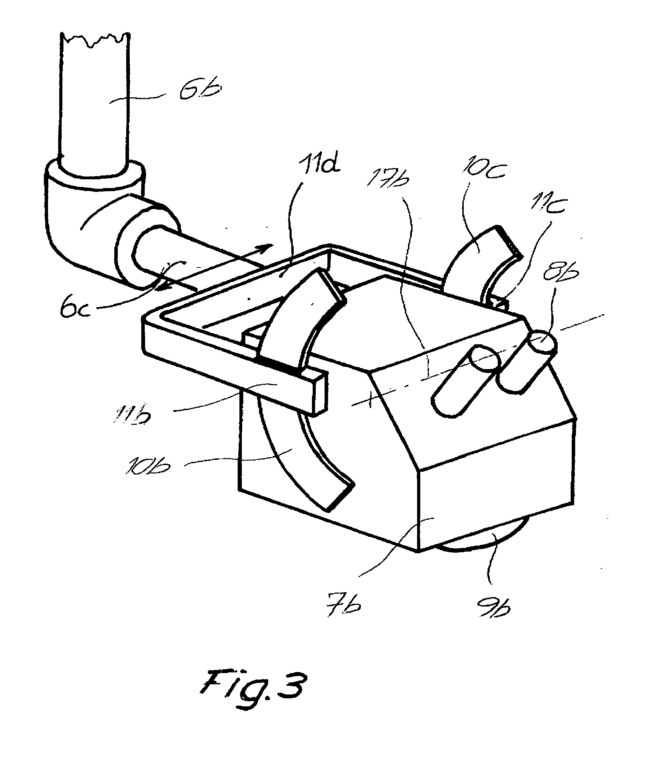

Über einen zwischen dem Optikträger 7a und dem Bogensegment 10a angeordneten A-B-Schlitten 12, 13 (Fig. 2) kann das Führungselement 10a gegenüber dem Optikträger 7a mittels Gewindespindeln 14, 15 verstellt werden. Der A-B-Schlitten kann als Variante auch zwischen dem Führungswagen 11 und dem Schwenkträger 6a angeordnet werden.

Das Schwenken des Optikträgers 7a gegenüber dem Schwenkträger 6a wird mittels einer auf das bogensegmentförmige Führungselement 10a einwirkenden und einstellbar ausgebildeten Bremse 16 reguliert.

Durch das Verstellen des bogensegmentförmigen Führungselementes 10a gegenüber dem Optikträger 7a, wird die (virtuelle) Schwenkachse 17a in den Schwerpunkt 18 des Optikträgers 7a verschoben. Dadurch kommt der Optikträger 7a in eine ausbalancierte Gleichgewichtslage, so dass er leichtgängig in eine beliebige Stellung verschwenkt werden kann und dann in dieser verbleibt.

In dem Ausführungsbeispiel der Fig. 3 sind zwei bogensegmentförmige Führungselemente 10b, 10c parallel zueinander angeordnet. Der Optikträger 7b weist einen Tubus 8b und ein Objektiv 9b auf und ist zwischen den Schenkeln des an seinem freien Ende als Gabel ausgebildeten Schwenkträgers 6b über die Führungselemente 10b, 10c am Führungswagen 11b, 11c gelagert. Diese Anordnung weist gegenüber einer einseitigen Lagerung wesentliche Vorteile auf, indem die Lagerkräfte reduziert werden. Die Führungselemente 10b, 10c können dabei als Gleit- oder Wälzlagerelemente ausgebildet sein. Mit einem analogen Verstellmechanismus, wie in Fig. 2 dargestellt, kann die Schwenkachse 17b in den Schwerpunkt des Optikträgers verschoben werden, so dass dieser ohne großen Kraftaufwand in eine beliebige Stellung bewegt wird und dann in dieser verbleibt. Eine Y-Verstellung (entlang der Schwenkachse 17a, b) ist durch eine nicht näher dargestellte, an sich bekannte Verstelleinrichtung zwischen einem Schwenkträgerteil 6c und einem Führungsteil 11d möglich (siehe Pfeil).

### Bezugszeichenliste

- 1: Ständerfuß
- 2: Ständersäule
- 3: Ausleger
- 4: Gelenk
- 5: Tragarm
- 6 a, b, c: Schwenkträger
- 7 a, b: Optikträger
- 8 a, b: Tubus
- 9 a, b: Objektiv
- 10 a, b, c: Bogensegmentförmiges Führungselement
- 11 a, b, c, d: Führungswagen
- 12: A-Schlitten
- 13: B-Schlitten (A-B-Schlitten in einer horizontalen und vertikalen Richtung)
- 14: Gewindespindel
- 15: Gewindespindel
- 16: Bremse
- 17 a, b: Schwenkachse
- 18: Schwerpunkt

## Patentansprüche

1. Operations-Mikroskop mit
einem Ständer (1, 2, 3, 4, 5),
einem Schwenkträger (6a, 6b),
einem über einen Führungswagen (11a, 11b) mit dem Schwenkträger (6a, 6b) beweglich gelagerten bogensegmentförmigen Führungselement (10a, 10b, 10c) und
einem an dem bogensegmentförmigen Führungselement (10a, 10b, 10c) befestigten Optikträger (7a, 7b) welcher durch Gleiten des bogensegmentförmigen Führungselements (10a, 10b, 10c) in dem Führungswagen (11a, 11b) um eine Achse (17a, 17b) geschwenkt werden kann, **dadurch gekennzeichnet, dass**
der Optikträger (7a, 7b) gegenüber dem bogensegmentförmigen Führungselement (10a, 10b, 10c) in zwei Raum-Achsen derart verstellbar ist, dass der Schwerpunkt des Optikträgers annähernd in die Schwenkachse des Optikträgers gebracht werden kann, wobei die Verstellung des bogensegmentförmigen Führungselements (10a, 10b, 10c) gegenüber dem Optikträger über lineare Schlitten (12, 13) erfolgt, die als A-B-Schlitten (12, 13) senkrecht zueinander angeordnet sind.

2. Operations-Mikroskop nach 1 **dadurch gekennzeichnet, dass** die Verstellung des bogensegmentförmigen Führungselementes (10a) mittels Gewindespindeln (14, 15) erfolgt.

3. Operations-Mikroskop nach einem der vorhergehenden Ansprüche, **dadurch gekennzeichnet, dass** am bogensegmentförmigen Führungselement (10a, 10b, 10c) ein Bremssystem (16) vorgesehen ist.

4. Operations-Mikroskop nach Anspruch 3, **dadurch gekennzeichnet, dass** das Bremssystem (16) eine mit dem bogensegmentförmigen Führungselement (10a, 10b, 10c) zusammenwirkende, feststellbare Zange aufweist.

5. Operations-Mikroskop nach einem der Ansprüche 3 oder 4, **dadurch gekennzeichnet, dass** das Bremssystem (16) eine regulier- und einstellbare Bremskraft aufweist.

6. Operations-Mikroskop nach einem der vorangehenden Ansprüche, **dadurch gekennzeichnet, dass** pro Schwenkachse (17a, 17b) jeweils zwei parallel zueinander angeordnete, bogensegmentförmige Führungselemente (10b, 10c) vorgesehen sind.

7. Operations-Mikroskop nach einem der vorangehenden Ansprüche, **dadurch gekennzeichnet, dass** das Führungselement (10) als gebogener Kugelführungsschlitten oder als gebogene Gleitführung aufgebaut ist.

8. Operations-Mikroskop nach einem der vorangehenden Ansprüche, **dadurch gekennzeichnet, dass** zwei parallel zueinander angeordnete Führungselemente (10b, 10c) vorgesehen sind.

## Claims

1. Surgical microscope having
a stand (1, 2, 3, 4, 5),
a swivelling arm (6a, 6b),
a guide element (10a, 10b, 10c), in the shape of an arcuate segment, mounted such that it can move together with the swivelling arm (6a, 6b) via a guide carriage (11a, 11b), and
an optics carrier (7a, 7b) which is fastened on the guide element (10a, 10b, 10c) in the shape of an arcuate segment and which can be swivelled about an axis (17a, 17b) by sliding the guide element (10a, 10b, 10c) in the shape of an arcuate segment in the guide carriage (11a, 11b), **characterized in that**
the optics carrier (7a, 7b) can be adjusted along two space axes with reference to the guide element (10a, 10b, 10c) in the shape of an arcuate segment in such a way that the centroid of the optics carrier can be brought approximately into the swivel axis of the optics carrier, the adjustment of the guide element (10a, 10b, 10c) in the shape of an arcuate segment relative to the optics carrier being performed via linear slides (12, 13) which are arranged in a mutually perpendicular fashion as A-B slides (12, 13).

2. Surgical microscope according to Claim 1, **characterized in that** the adjustment of the guide element (10a) in the shape of an arcuate segment is performed by means of threaded spindles (14, 15).

3. Surgical microscope according to one of the preceding claims, **characterized in that** a brake system (16) is provided on the guide element (10a, 10b, 10c) in the shape of an arcuate segment.

4. Surgical microscope according to Claim 3, **characterized in that** the brake system (16) has a lockable gripping device cooperating with the guide element (10a, 10b, 10c) in the shape of an arcuate segment.

5. Surgical microscope according to one of Claims 3 or 4, **characterized in that** the brake system (16) exhibits a braking force which can be regulated and set.

6. Surgical microscope according to one of the preceding claims, **characterized in that** two guide elements (10b, 10c) in the shape of an arcuate segment which are arranged parallel to one another are provided in each case per swivel axis (17a, 17b).

7. Surgical microscope according to one of the preceding claims, **characterized in that** the guide element (10) is constructed as arcuate ball-bearing slides or as an arcuate slideway.

8. Surgical microscope according to one of the preceding claims, **characterized in that** two guide elements (10b, 10c) arranged parallel to one another are provided.

## Revendications

1. Microscope d'opération, qui présente
un socle (1, 2, 3, 4, 5),
un support pivotant (6a, 6b),
un élément de guidage (10a, 10b, 10c) en forme de segment d'arc de cercle monté de manière à pouvoir se déplacer avec le support pivotant (6a, 6b) par l'intermédiaire d'un chariot de guidage (11a, 11b) et
un support d'optique (7a, 7b) fixé sur l'élément de guidage (10a, 10b, 10c) en forme de segment d'arc de cercle et qui peut pivoter autour d'un axe (17a, 17b) par coulissement de l'élément de guidage (10a, 10b, 10c) en forme de segment d'arc de cercle dans le chariot de guidage (11a, 11b),
**caractérisé en ce que**
le support d'optique (7a, 7b) peut être déplacé par rapport à l'élément de guidage (10a, 10b, 10c) en forme de segment d'arc de cercle suivant deux axes spatiaux, de telle sorte que le centre de gravité du support d'optique puisse être amené approximativement sur l'axe de pivotement du support d'optique, le déplacement de l'élément de guidage (10a, 10b, 10c) en forme de segment d'arc de cercle par rapport au support d'optique s'effectuant par des glissières linéaires (12, 13) qui sont disposées perpendiculairement l'une à l'autre sous forme de glissières A-B (12, 13).

2. Microscope d'opération selon la revendication 1, **caractérisé en ce que** le déplacement de l'élément de guidage (10a) en forme de segment d'arc de cercle s'effectue à l'aide de tiges filetées (14, 15).

3. Microscope d'opération selon l'une quelconque des revendications précédentes, **caractérisé en ce qu'**un système de freinage (16) est prévu sur l'élément de guidage (10a, 10b, 10c) en forme de segment d'arc de cercle.

4. Microscope d'opération selon la revendication 3, **caractérisé en ce que** le système de freinage (16) présente une pince qui peut être fixée et qui coopère avec l'élément de guidage (10a, 10b, 10c) en forme de segment d'arc de cercle.

5. Microscope d'opération selon la revendication 3 ou 4, **caractérisé en ce que** la force de freinage du système de freinage (16) peut être régulée ou réglée.

6. Microscope d'opération selon l'une quelconque des revendications précédentes, **caractérisé en ce que** pour chaque axe de pivotement (17a, 17b), il présente deux éléments de guidage (10b, 10c) en forme de segment d'arc de cercle et disposés parallèlement l'un à l'autre.

7. Microscope d'opération selon l'une quelconque des revendications précédentes, **caractérisé en ce que** l'élément de guidage (10) est construit comme coulisse de guidage cintrée en sphère ou comme guide courbe de coulissement.

8. Microscope d'opération selon l'une quelconque des revendications précédentes, **caractérisé en ce qu'**il présente deux éléments de guidage (10b, 10c) disposés parallèlement l'un à l'autre.
